# EUROPEAN PATENT SPECIFICATION

(11) **EP 2 978 403 B1**
(45) Date of publication and mention of the grant of the patent: **03.07.2019**
(21) Application number: 13732261.6
(22) Date of filing: 29.03.2013
(51) Int. Cl.: A61K 9/00, A61K 9/06, A61K 9/14, A61K 31/58

(54) **ORAL SUSPENSION FOR TREATING EOSINOPHILIC ESOPHAGITIS**
ORALE SUSPENSION ZUR BEHANDLUNG VON EOSINOPHILER ÖSOPHAGITIS
SUSPENSION ORALE POUR TRAITER UNE SOPHAGITE ÉOSINOPHILE

(43) Date of publication of application: 03.02.2016
(73) Proprietor: OCEAN FARMA S.r.l, 04100 Latina (IT)
(72) Inventor: ANNALORO, Raffaele, 00040 Pomezia (IT); SONAGLIA, Achille, 00040 Pomezia (IT)
(74) Representative: Cioncoloni, Giuliana
(86) International application number: PCT/IT2013/000096
(87) International publication number: WO 2014/155397

(56) References cited:
- US-A1- 2007 020 299
- US-A1- 2009 191 275
- US-A1- 2010 216 754

## Description

This invention relates to a pharmaceutical formulation of a stable, high-viscosity corticosteroid, more specifically budesonide, suspension to be administered orally for the treatment of esophageal pathologies of immune origin and more specifically for the treatment of eosinophilic esophagitis, or esophageal eosinophilia, in both children and adults.

Eosinophilic esophagitis is a recently recognized disorder characterized by the accumulation of eosinophils or eosinophil granulocytes, i.e. white blood cells constituting one of the immune system components, in the esophagus. Symptoms of eosinophilic esophagitis frequently mimic those of gastroesophageal reflux disease, but the two diseases are quite different in terms of the histopathology and response to therapy. Eosinophilic esophagitis is an increasingly recognized cause of dysphagia and possibly heartburn that is unresponsive to antireflux measures. The annual incidence of eosinophilic esophagitis in children has been estimated to be in the order of 1:100,000, which makes this disease to be classified as a rare disease and the treatment to be termed as 'orphan'.

A number of treatments were demonstrated effective in eliminating symptoms and reducing esophageal eosinophilia, each carrying its own risks and benefits and ease of compliance (i.e. the adherence of the patient to the medical therapy). Dietary elimination is safe and offers lifelong treatment, but compliance may be difficult. Topical steroids offer a valid alternative. Patients swallow a steroid based oral formulation, thus delivering a topical anti-inflammatory agent directly to the esophageal mucosa. Corticosteroids inhibit the synthesis and secretion of cytokines, known to influence eosinophil growth, differentiation and activation. Especially children, who are unresponsive to dietary restriction, or cannot tolerate a dietary treatment, clearly may benefit from orally administered corticosteroids. Indeed, swallowed topical steroids were shown to induce and maintain low esophageal eosinophil levels.

Administration of oral formulations of budesonide turns out to be particularly suitable for treating eosinophilic esophagitis because of its high local activity. Compared with beclomethasone dipropionate, the topical potency of budesonide is two-fold greater, and greater yet if compared with prednisone. Budesonide, administered orally, has an extensive first-pass metabolism in the liver with a residual bioavailability of only 11%. Budesonide is eliminated with the urine and faeces in the form of its metabolites that are endowed with only a very slight corticosteroid activity.

Recently, a successful treatment of eosinophilic esophagitis was reported with the use of an oral viscous suspension of budesonide. In this trial children under the age of ten years received 1 mg/day of an oral viscous budesonide liquid suspension obtained by mixing just before administration two 0.5 mg/2 ml Pulmicort Respules; a water suspension of budesonide meant for inhalation purposes for treating asthma; with 5 grams of sucralose. Only one patient received double this dose.

An ideal formulation for an oral suspension is one that prolongs the contact of the active ingredient with the mucous membranes of the esophagus.

Document US 2010/216754 A1 (MALCOLM HILL), published on 30th August, 2010, having the title "COMPOSITIONS FOR THE TREATMENT OF INFLAMMATION OF THE GASTROINTESTINAL TRACT", discloses a suspension containing budesonide and excipients that increase the viscosity of the composition. However, it is directed to treating gastroesophageal reflux disease (GERD) and no ground may be found therein for a formulation containing budesonide and viscosity-enhancing excipients that is suitable for treating eosinophilic esophagitis.

Document US 2009/191275 A1 (RANJAN DOHIL), published on 30th July, 2009, having the title "VISCOUS BUDESONIDE FOR THE TREATMENT OF INFLAMMATORY DISEASES OF THE GASTRONITESTINAL TRACT", discloses a suspension containing budesonide and viscosity-enhancing agents such as sorbitol, PVP, microcrystalline cellulose for the treatment of eosinophilic esophagitis.

Document US 2007/020299 A1 (JAMES D. DIPKIN), published on 25th January, 2007, having the title "INHALANT FORMULATION CONTAINING SULFOALKYL ETHER CYCLODEXTRIN AND CORTICOSTEROID" discloses an inhalable composition comprising budesonide. The suspension comprises excipients such as sodium citrate, citric acid, disodium EDTA, polysorbate and water.

International Application published under the Patent Cooperation Treaty at No. WO 2009/132048 A2, having the title "TREATING EOSINOPHILIC ESOPHAGITIS", of Meritage Pharma, Inc. at San Diego, California, U.S.A., referred to as Document D1 hereinafter, discloses methods and compositions suitable for treating eosinophilic esophagitis. More specifically, D1 discloses methods that include orally administering a steroid, such as budesonide, together with a mucoadherent to a mammal such that eosinophilic esophagitis is treated, where a mucoadherent can be any compound that prolongs the contact of another molecule, such as a steroid, to mucous membranes, e.g. to coat the surface of the esophagus. Compositions are disclosed in D1 containing a steroid and a mucoadherent. A delivery system is also disclosed in D1 including a syringe containing a composition having a steroid and a mucoadherent. The delivery system can contain a tube that allows a user to deliver the composition to the back of the patient's oral cavity.

Hyaluronic acid is envisaged in D1, which, however, is not only an excipient, but also an active principle.

The tube indicated as an advantage in D1, actually has the great disadvantage that a child cannot self-administer it and therefore it is necessary that an expert adult shall perform the not easy task of using the delivery device without irritating the patient. Moreover, such a complex manoeuvre may compromise compliance, which is an extremely important factor for this type of chronic patient.

The object of this invention is to provide a pharmaceutical formulation of a stable budesonide high-viscosity oral anti-inflammatory suspension for treating eosiniphilic esophagitis, that manages to penetrate esophageal wall and reaches eosinophiles with a steady absorption, formulated with ingredients of well established medicinal use. The oral suspension is easily administered and even easily self-administered, thereby ensuring best compliance.

Therefore, it is the subject-matter of this invention the formulation of appended Claim 1.

This invention will be understood based on the following detailed disclosure of ways for formulating and carrying out it, only given as a matter of example, absolutely not of restriction.

Possible thickening agents that were taken into consideration were:
- Hypromellose (hydroxypropylmethylcellulose): this one is present in Aircort™ nasal spray, it is used as a thickening agent in ophthalmic preparations, but not in oral suspension;
- Methylcellulose: this one is recommended as a thickening agent in sugar-based suspension formulations, but it cannot be used because of its incompatibility with parabens;
- microcrystalline cellulose / sodium carboxymethylcellulose (Avicel RC-591) already present in Peridon™ suspension, but it is primarily a suspending agent, rather than a thickening agent;
- PVP (polyvinylpyrrolidone): this one is recommended as a thickening agent in oral suspensions and solutions.

Placebo testing for identification of the optimal viscosity comprised:
- Hypromellose (ref.: RAYMOND C. ROWE (ed.), Handbook of Pharmaceutical Excipients, Royal Pharmaceutical Society, UK, 7th ed.);
- microcrystalline cellulose (89% by weight)/sodium carboxymethylcellulose (11% by weight) mixture (commercially available as Avicel RC-591) (ref. : *http:llwww.fmcbiopolymer. com*/*Pharmaceutical*/*Products*/*Avicelforsuspensions.aspx*);
- Polyvinylpyrrolidone (PVP K90) (ref.: *Handbook of Pharmaceutical Excipients* cit.).

One possible formulation would comprise a combination of the above mentioned excipients at various concentrations.

The present invention envisages a formulation where ascorbic acid and xylitol replace sorbitol:

**Formulation (a)**

| **Ingredient** | **%weight-volume** |
|---|---|
| Budesonide | 0.018-0.022 |
| Polysorbate | 0.0018-0.0022 |
| Disodium edetate (EDTA) | 0.009-0.011 |
| Xylitol | 28.62-34.36 |
| microcrystalline cellulose (89% by weight)/sodium carboxymethylcellulose (11% by weight) mixture (Avicel RC-591) | 1.08-1.32 |
| Polyvinylpyrrolidone PVP K90 | 9.0-11.0 |
| Potassium sorbate | 0.18-022 |
| Sodium citrate dihydrate | 0.0045-0.0055 |
| Ascorbic acid | 0.09-0.11 |
| Citric acid monohydrate | *q.s.* to pH 4.5 |
| Highly purified water | *q.s.* to 100 ml |

Pilot batches of Formulation *(a)* were manufactured in 100 ml amber glass bottles and put under stability investigation according to ICH recom-mended conditions. All above formulations appear to be stable and suitable for clinical investigation.

Specific ways of formulating and carrying out this invention have been disclosed, but it is to be understood that the scope of protection thereof is only restricted by the appended claims.

## Claims

1. A formulation of a stable budesonide high-viscosity oral anti-inflammatory suspension, having the composition:
| **Ingredient** | **%weight-volume** |
|---|---|
| Budesonide | 0.018-0.022 |
| Polysorbate | 0.0018-0.0022 |
| Disodium edetate (EDTA) | 0.009-0.011 |
| Xylitol | 28.62-34.36 |
| Formulation (A) | 1.08-1.32 |
| Polyvinylpyrrolidone PVP K90 | 9.0-11.0 |
| Potassium sorbate | 0.18-022 |
| Sodium citrate dihydrate | 0.0045-0.0055 |
| Ascorbic acid | 0.09-0.11 |
| Citric acid monohydrate | *q.s.* to pH 4.5 |
| Highly purified water | *q.s.* to 100 ml |
suitable for the treatment of eosiniphilic esophagitis.
with formulation (A): microcrystalline cellulose /sodium carboxymethylcellulose mixture (Avicel RC-591).

## Patentansprüche

1. Formulierung einer stabilen hochviskosen oralen entzündungshemmenden Budesonid-Suspension mit der Zusammensetzung:
| **Inhaltsstoff:** | **%weight-volume** |
|---|---|
| Budesonid | 0,018-0,022 |
| Polysorbat | 0,0018-0,0022 |
| Dinatriumedetat (EDTA) | 0,009-0,011 |
| Xylitol | 28,62-34,36 |
| Formulierung (A) | 1,08-1,32 |
| Polyvinylpyrrolidon PVP K90 | 9,0-11,0 |
| Kaliumsorbat | 0,18-022 |
| Natriumcitratdihydrat | 0,0045-0,0055 |
| Ascorbinsäure | 0,09-0,11 |
| Citronensäure-Monohydrat | q.s. bis pH 4. 5 |
| Hochreines Wasser | q.s. bis 100 ml |
geeignet zur Behandlung von eosinophiler Ösophagitis.
mit der Formulierung (A): mikrokristalline Cellulose/Natriumcarboxymethylcellulose-Gemisch (Avicel RC-591)

## Revendications

1. Une formulation d'une suspension anti-inflammatoire orale de budésonide stable à haute viscosité, ayant la composition:
| **Ingrédient** | **%poids-volume** |
|---|---|
| Budésonide | 0.018-0.022 |
| Polysorbate | 0.0018-0.0022 |
| Edétate disodique (EDTA) | 0.009-0.011 |
| Xylitol | 28.62-34.36 |
| Formulation (A) | 1.08-1.32 |
| Polyvinylpyrrolidone PVP K90 | 9.0-11.0 |
| Sorbate de potassium | 0.18-022 |
| Citrate de sodium dihydraté | 0.0045-0.0055 |
| Acide ascorbique | 0.09-0.11 |
| Acide citrique monohydraté | q.s. à pH 4,5 |
| Eau hautement purifiée | q.s. à 100 ml |
Avec formulation (A): mélange de cellulose microcristalline / carboxyméthylcellulose sodique (Avicel RC-591).
